Europäisches Patentamt

(19) **European Patent Office**

**Office européen des brevets**

(11) Publication number: **0 015 721**
**A1**

(12) # EUROPEAN PATENT APPLICATION

(21) Application number: **80300601.4**

(22) Date of filing: **28.02.80**

(51) Int. Cl.³: **A 61 K 31/49**
// (A61K31/49, 31/375)

(30) Priority: **28.02.79 GB 7907097**

(43) Date of publication of application: **17.09.80**
**Bulletin 80/19**

(84) Designated Contracting States: **AT BE CH DE FR GB IT LU NL SE**

(71) Applicant: **Fox, Edward Solomon, The Fox Inn Dunsmore, Aylesbury Bucks. HP22 6QH (GB)**

(72) Inventor: **Fox, Edward Solomon, The Fox Inn Dunsmore, Aylesbury Bucks. HP22 6QH (GB)**

(74) Representative: **BAYLISS, Geoffrey Cyril et al, BOULT, WADE & TENNANT 27 Furnival Street, London EC4A 1PQ (GB)**

(54) Composition for rheumatoid arthritis and other arthritic conditions and method for its preparation.

㊻ This invention relates to pharmaceutical compositions for the treatment of rheumatoid arthritis and other arthritic conditions in humans, as well as various canine complaints.

The composition includes quinine sulphate and ascorbic acid. The quinine sulphate constitutes between 3.5 percent and 18 percent inclusive by weight of the composition. The composition is preferably in tablet form.

EP 0 015 721 A1

COMPOSITION FOR RHEUMATOID ARTHRITIS AND OTHER ARTHRITIC
CONDITIONS AND METHOD FOR ITS PREPARATION

This invention relates to pharmaceutical (including veterinary) compositions for the treatment of rheumatoid arthritis and other arthritic conditions.

According to the present invention there is provided a pharmaceutical composition in the form of a solid mixture comprising quinine sulphate and ascorbic acid.

As used herein, the term "quinine sulphate" is intended to mean any compound including the quinine and sulphate species, and preferably quinine bisulphate.

Quinine sulphate may preferably constitute between 3.5 percent and 18 percent inclusive by weight of the composition. The composition is preferably constituted in unit dosage form, for example in tablet form, in which the amount of quinine sulphate per unit dose is not greater than 35 milligrams.

Rheumatoid arthritis and other arthritic complaints may be caused by crystalline material, which is formed in the human body, and which can concentrate at points in the body whereby it applies pressure to nerves causing acute pain or even a form of paralysis.

It is believed that the pharmaceutical composition of the present invention serves to relax the muscles of the patient, hence reducing the pressure caused by the existence of the crystalline formation, and simultaneously destroys the crystalline formation.

Tablets according to the invention may be produced by conventional tabletting techniques, and may include conventional additives, such as inert fillers and diluents, binders and flavourings.

Individual tablets may preferably weigh from 100 milligrams to 1 gram, of which from 18 to 35 milligrams may preferably be constituted by quinine sulphate. The amount of ascorbic acid is not particularly critical provided that sufficient is present, as the body will excrete any excess, but it is generally preferred that the amount of the ascorbic acid is from 4 to 25 times that of quinine sulphate by weight. In a particularly preferred embodiment, no filler is used, and ascorbic acid constitutes the balance of a tablet of which from 3.5 to 18% is quinine sulphate.

The composition according to the invention may be administered to mammalian animals, particularly humans and dogs, in the treatment of arthritic conditions, for example rheumatoid arthritis.

Example

Quinine sulphate and ascorbic acid were ground together in a mortar and pestle in the ratio 35 to 165 by weight, and the resulting dry mixture was formulated into 200 milligram tablets, of which 35 milligrams was constituted by quinine, the balance being ascorbic acid. The tablets were found to be highly efficacious in the treatment of rheumatoid arthritis in human beings.

They were also found to be useful in the treatment of arthritis, sometimes wrongly diagnosed as "slipped disc" in dogs.

CLAIMS

1. A pharmaceutical composition in the form of a solid mixture comprising quinine sulphate and ascorbic acid.

2. A pharmaceutical composition as claimed in claim 1 wherein the quinine sulphate constitutes from 3.5 to 18 by weight of the composition.

3. A composition as claimed in claim 2 in unit dosage form the amount of quinine sulphate in each dose forms not greater than 35 milligrams.

4. A composition as claimed in claim 3, in tablet form.

5. A pharmaceutical tablet including a pharmaceutically effective amount not exceeding 3.5 m.g. of quinine sulphate, the balance being essentially ascorbic acid.

6. A method of making a pharmaceutical composition, which method comprises mixing quinine sulphate and ascorbic acid to produce a composition as defined in any one of claims 1 to 4.

| Category | Citation of document with indication, where appropriate, of relevant passages | Relevant to claim |
|---|---|---|
| | **DOCUMENTS CONSIDERED TO BE RELEVANT** | |
| X | ROTE LISTE 1961, Editio Cantor, Aulendorf/Württ page 181, "Chinin-Redoxon" -- | 1-6 |
| X | DICTIONNAIRE VIDAL 1961, Office de Vulgarisation Pharmaceutique, Paris VIII, page 1388, "Quinine Vitamine-C Grand", dragées et suppositoires -- | 1-6 |
| X | ROTE LISTE 1965, Editio Cantor, Aulendorf/Württ page 1291, "Ypsichin Dragees" -- | 1-6 |
| X | DICTIONNAIRE VIDAL 1967, O.V.P., Paris VIII page 107, "Asphogan à la Mépyramine et à la Vitamine C" ---- | 1-6 |

**CLASSIFICATION OF THE APPLICATION (Int. Cl. 3)**

A 61 K 31/49//
(A 61 K 31/49
31/375)

**TECHNICAL FIELDS SEARCHED (Int.Cl. 3)**

A 61 K 31/49

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 15-04-1980 | BRINKMANN |